# EUROPEAN PATENT SPECIFICATION

(11) **EP 2 477 562 B1**
(45) Date of publication and mention of the grant of the patent: **31.12.2014**
(21) Application number: 10754496.7
(22) Date of filing: 15.09.2010
(51) Int. Cl.: A61B 17/24, A61B 17/04, A61B 19/00

(54) **PHARYNX PROTECTOR**
SCHUTZ FÜR PHARYNX
PROTECTEUER PHARYNGEAL

(30) Priority: 18.09.2009 SE 0950683
(43) Date of publication of application: 25.07.2012
(73) Proprietor: Atos Medical AB, 242 22 Hörby (SE)
(72) Inventor: NILSSON, Andreas, S-227 83 Lund (SE); PERSSON, Jan-Ove, S-243 95 Höör (SE)
(74) Representative: Ström & Gulliksson AB
(86) International application number: PCT/EP2010/063519
(87) International publication number: WO 2011/032965

(56) References cited:
- WO-A1-97/41807
- WO-A1-2005/089846
- WO-A1-2007/018472
- WO-A2-2007/017447
- RU-C1- 2 187 252
- US-A- 5 078 743

## Description

### Field of the Invention

This invention pertains in general to the field of a pharynx protector, for protecting the oesophagus and/or pharynx during performance of a secondary puncture in a laryngectomized patient, said pharynx protector having a proximal end and a distal end, said distal end being adapted to be inserted into the oesophagus of a patient, said pharynx protector comprising a proximally located elongated tubular part and a distally located receiving portion.

### Background of the Invention

In the field of laryngectomy a voice prosthesis is often used for hacheoesophagal speech. The voice prosthesis is then placed in a puncture in the tracheoesophageal wall. The voice prosthesis may be placed in said puncture when the voice box is removed and the trachea is sutured to the skin of the neck - so called primary puncture - or after time of surgery, under general anesthesia - so called secondary puncture. A voice prosthesis has a tubular body, with a flange in each end. The tubular body is to be mounted in the tracheoesophageal wall with a flange situated on the tracheal side, substantially hindering movement of the voice prosthesis into the oesophagus, and the other flange situated on the oesophagal side, hindering movement of the voice prosthesis into the trachea. A valve member is located in the lumen of the tubular body. The voice prosthesis may also be provided with a safety strap, arranged on the flange intended to be situated on the tracheal side.

Laryngectomy may for example be performed in cases of laryngeal cancer.

To create a tracheoesophageal puncture during primary puncture, a pharynx protector is inserted caudal in the pharynx until the tip of the pharynx protector reaches the intended puncture site in the esophagus. The pharynx protector is a hollow, rigid, and cylindrical device with a handle. It is inserted in the pharynx/esophagus to protect the posterior wall during puncture. The tip of the device has normally an oblique opening, which is palpated by the surgeon to verify the correct position for puncture. The device has a slot running from the oblique opening along the top of the cylindrical part to enable radial puncturing for surgeons who prefer doing so. The tip of the pharynx protector is palpated through the trachea to verify the correct placement of the puncture. The puncture is made with a puncture needle through the tracheoesophageal wall against the pharynx protector. The puncture needle is a thick and hollow needle normally made of steel. The needle is used to create the puncture and to facilitate the subsequent introduction of a guide wire, which is inserted through the puncture needle.

The guide wire is normally a plastic thread, which is plastically deformable. The puncture needle may have a bent tip in order to direct the guide wire into the hollow cylindrical part of the pharynx protector. The puncture needle is oriented so that a bent tip thereof directs a guide wire - subsequently inserted through the puncture needle - into the lumen of the pharynx protector. Next, the guide wire is introduced through the puncture needle until the distal tip of the guide wire extends approximately 20 cm through the pharynx protector. The puncture needle and the pharynx protector are removed, leaving the guide wire in place through the puncture of the tracheoesophageal wall. Thereafter, a voice prosthesis is arranged on the guidewire and pulled through the tracheoesophageal puncture.

During secondary puncture a rigid esophagoscope is generally inserted in the esophagus instead of a pharynx protector until the tip of the esophagoscope can be palpated at the puncture site. The puncture is then made with the puncture needle against the esophagoscope which acts as a pharynx protector.

US 6,159,243 discloses a voice prosthesis implantation kit for secondary puncture including; a pharynx protector in form of a leader element, which can be introduced via the mouth to the location where the voice prosthesis is to be implanted. The leader element comprises an expansion element in form of a balloon, which balloon shall interact with a cutting element carrying a dilator for a voice prosthesis. The kit according to US 6,159,243 is only usable for secondary puncture. However, the pharynx protector according to US 6,159,243 is accompanied buy several drawbacks. For example, this pharynx protector comprises several parts, and a complicated configuration of these parts, such as the need of a pressure applying device for expanding the balloon; the positioning of the cutting device is unguided, and has to be exactly positioned within the balloon, not to risk that the back wall of the oesophagus from being penetrated.

WO 2005/089846 discloses a device for selectively isolating one of a pair of bronchi or lungs. WO 2007/018472 discloses a device for facilitating tracheotomy, comprising two branches.

Hence, an improved pharynx protector would be advantageous, and in particular a pharynx protector allowing for use in secondary puncture; said pharynx protector being easy to manufacture and assemble, leading to a lower production cost, providing a safe way to penetrate the front wall of the oesophagus without risking injuring other parts of the oesophagus, and providing a pharynx protector that is more pleasant for the patient during insertion into the oesophagus, would be advantageous.

### Summary of the Invention

Accordingly, the present invention preferably seeks to mitigate, alleviate or eliminate one or more of the above-identified deficiencies in the art and disadvantages singly or in any combination and solves at least the above mentioned problems by providing a pharynx protector for protecting the oesophagus and/or pharynx during performance of a laryngectomy, said pharynx protector having a proximal end and a distal end, said distal end being adapted to be inserted into the oesophagus of a patient, said pharynx protector comprising an elongated tubular part and a distally located receiving portion, wherein said tubular part being flexible, and said receiving portion comprising; an opening, communicating with the lumen of the tubular part, such that a guide wire may be inserted through the opening and further passed proximally out of the pharynx protector; and a surface of the inner wall, having a normal passing through said opening, being of a rigid material.

Advantageous features hereof are embodied in the dependent claims.

### Brief Description of the Drawings

These and other aspects, features and advantages of which the invention is capable of will be apparent and elucidated from the following description of embodiments of the present invention, reference being made to the accompanying drawings, in which
Figs. 1a to 1d illustrate a pharynx protector according to one embodiment of the present invention;
Figs.2a to 2c illustrate a pharynx protector according to a second embodiment of the present invention; and
Figs.3a to 3c illustrate a pharynx protector according to a third embodiment of the present invention.

### Description of embodiments

The following description focuses on embodiments of the present invention applicable to a pharynx protector for receiving a puncture needle during puncture of the tracheoesophageal wall. The needle is used to create the puncture and to facilitate the subsequent introduction of a guide wire, which is inserted through the puncture needle, for example to insert a voice prosthesis in the tracheoesophageal wall by the aid of a dilator. A guidewire-dilator-prosthesis assembly is then pulled through the puncture site, dilating the puncture to facilitate the subsequent prosthesis placement.

For this purpose a pharynx protector for protecting the oesophagus and/or pharynx during performance of a laryngectomy is provided. The pharynx protector has a proximal end and a distal end, said distal end being adapted to be inserted into the oesophagus of a patient. The pharynx protector comprises an elongated tubular part and a distally located receiving portion, for receiving a puncture needle during laryngectomy. The tubular part is flexible, and the receiving portion comprises an opening, communicating with the lumen of the tubular part, such that a guide wire may be inserted through the opening and further passed proximally out of the pharynx protector. The surface of the inner wall of the receiving portion, having a normal passing through said opening, is of a rigid material.

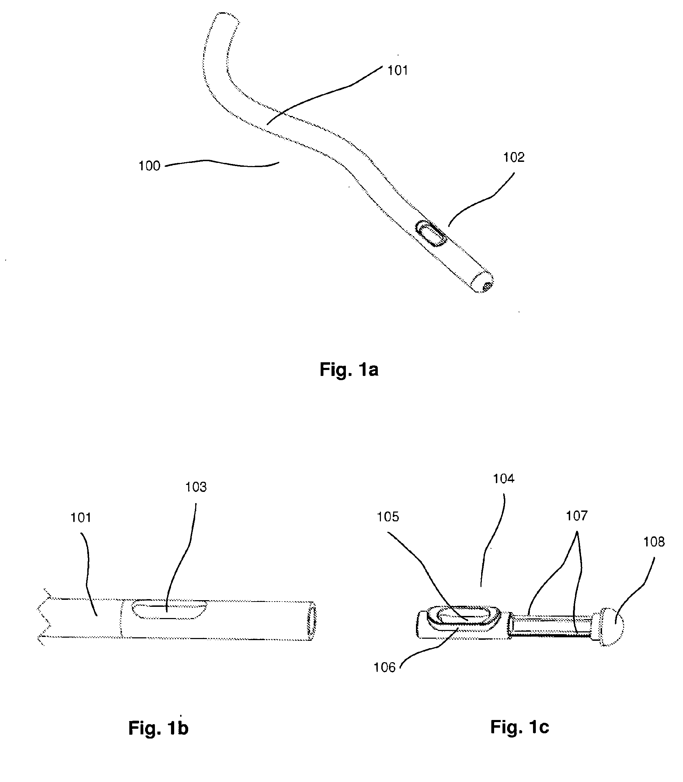

During secondary puncture, i.e. when a total laryngectomy has been performed without a voice prosthesis being placed at the time of surgery, a pharynx protector **100,** comprising a flexible tube **101,** according to Fig. 1a, is inserted into the oesophagus of the patient. The tube **101** is flexible to facilitate insertion of the tube **101** in the oesophagus of the patient. To facilitate use and decrease discomfort to the patient the tube **101** should have a flexability sufficient to exit the mouth of the patient while still extending down into oesophagus, without too strong bending having to be resisted by the body of the patient.

Close to a distal end of the flexible tube **101,** the pharynx protector **100** is provided with a receiving portion **102,** for receiving the puncture needle after said puncture needle has penetrated the tracheoesophageal wall.

The receiving portion **102** comprises an opening **103** in the tubular wall of the flexible tube **101,** in accordance with Fig. 1b. The distal end of the flexible tube **101** is the end of the pharynx protector that is inserted through the mouth of the patient.

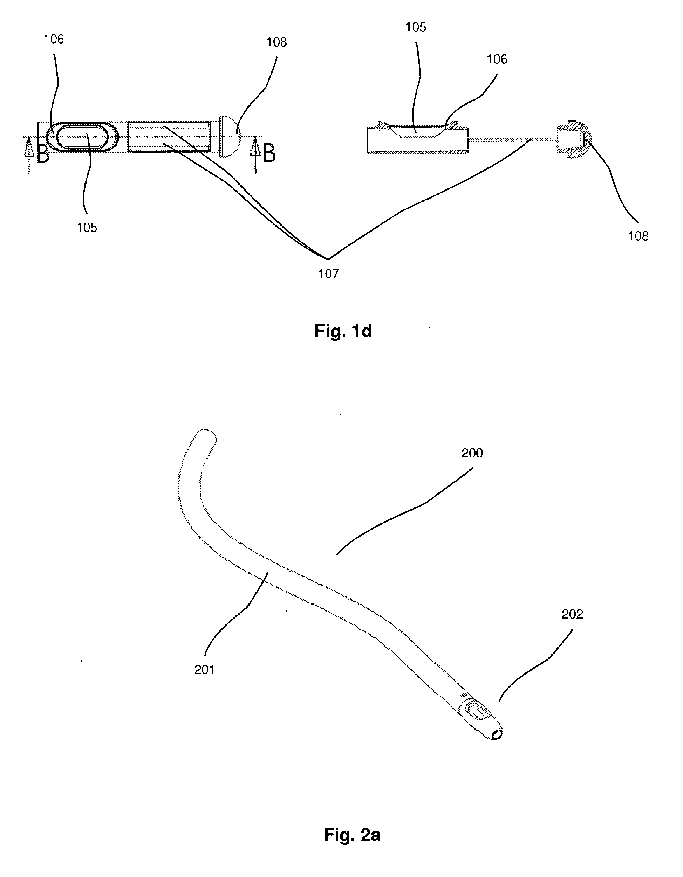

The receiving portion further comprises a rigid tubular element **104,** inserted into the lumen of the flexible tube **101,** in accordance with Fig. 1c. Fig. 1d illustrates a cross section along a plane B. The rigidity of the tubular element **104** should be such that it a puncture needle cannot penetrate through the wall of the tubular element **104** during practical circumstances. Thus, the puncture needle cannot penetrate the back wall of the tubular element **104,** whereby the back wall of the oesophagus is protected.

The tubular element **104** has an opening **105** in the tubular wall thereof. The opening **105** corresponds in shape and size to the opening **103** in the tube **101.** The opening **105** may have a circumferential rim **106** on the outside surface of the tubular element **104.** This rim **106** may be adapted to securingly fit within the opening **103,** such that the tubular element **104** may be snap fitted to the tube **101** by insertion through the distal end of the tube **101.** In this way, the tubular element **104** may be secured to the tube **101** and thereby the pharynx protector **100.**

The lumen of the tubular element **104,** when inserted in the tube **101,** communicates with the lumen of the tube **101,** such that a guide wire may be inserted through the openings **103** and **105** and further passed proximally out of the pharynx protector and thereby out of the mouth of the patient.

The openings **103** and **105** may have an oval shape, which will increase the target area for the puncture needle penetrating the tracheoesophagal wall.

In the distal end of the tubular element **104,** when arranged in the tube **101,** the tubular element **105** may be provided with extension elements, in form of bars **107,** extending coaxially with the central axis of the tubular element **104** in a distal direction. The bars **107** then corresponds to the length between the distal end of the opening **103** in the tube **101** and the distal end of the tube **101.** In the distal end of the bars **107** a tip element **108** may be provided, such that the distal opening of the tube **101** is closed when the tubular element **104** is inserted into the tube **101.**

The outer surface of the tip element **108** may be rounded, such as semi-spherical or dome shaped. Such shape will facilitate insertion of the pharynx protector through the mouth of the patient, and also facilitate the travel through the oesophagus of the patient to the target site. The tip element **108** may also be provided with an axial opening, having a direction axial to the central axis of the tube **101** and the tubular element **104.** This axial opening may be used for inspection with a flexible endoscope. The axial opening may also be used if a guide wire is used for insertion of the pharynx protector, making it easier to introduce the pharynx protector in a difficult throat with strictures and other anatomical defects.

The bars **107** may be of such dimensions as to allow a certain flexibility, even though manufactured in the same rigid material as the rest of the tubular element **104.** When the tubular element **104** is provided with bars **107,** the distal part of the pharynx protector will be more flexible compared to the one lacking such extension elements, leading to decreased inconvenience during insertion of the pharynx protector. Also, the tubular element **104** and tip element **108** may be manufactured as one piece, making the pharynx protector cheaper, compared to having a separate tip element **108.**

It is however possible to omit the extension elements **107,** whereby the tip element **108** is arranged at the end of the tubular element **104.** Thus, the extension elements **107** have been replaced by an extended part of the tubular element **104.** In this way the tubular element **104** will form a barrel with an opening **105** in the tubular part of the barrel. Of course, the rest of the features relating to Figs. 1a to 1d are equally applicable to the barrel, without departing from the scope of the invention.

In another embodiment, according to Figs. 2a to 2c, a pharynx protector 200 comprises a flexible tube 201, provided with a receiving portion 202 at the distal end thereof. The receiving portion 202 is a portion for receiving a puncture needle after said puncture needle has penetrated the tracheoesophageal wall.

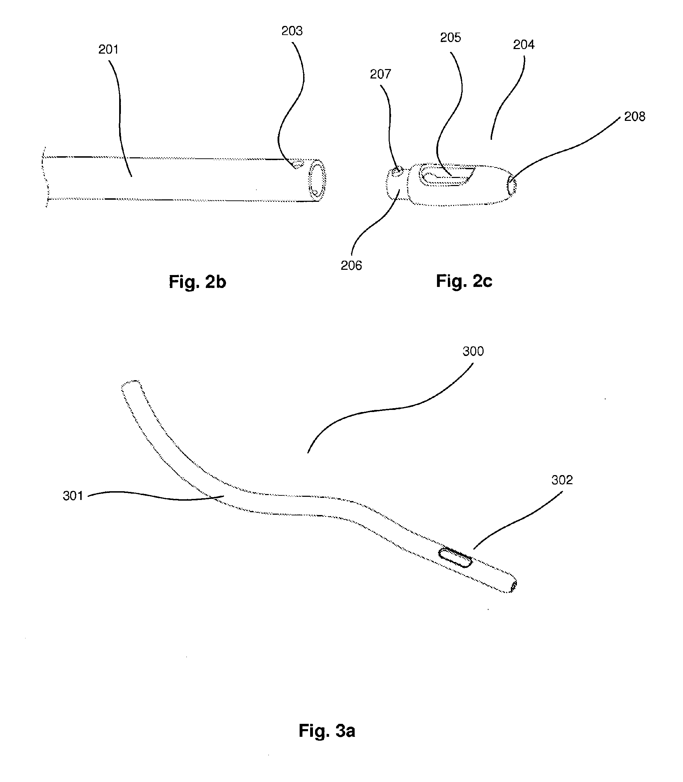

The tube 201 is provided with two or more openings 203, in accordance with Fig. 2b, in the distal end thereof. The openings 203 constitute an attaching means, for securingly attaching a rigid tubular element 204 to the tube 201.

A receiving portion 202 comprises an opening 205 in the tubular wall of the tubular element **204,** in accordance with Fig. 2c. The opening **205** is adapted in shape and size for reception of a puncture needle after the puncture needle has penetrated/punctured the tracheoesophageal wall, in conformity with the openings **103** and **105** in respect of the embodiment disclosed in Figs. 1a to 1d.

The opening **205** may have an oval shape, which will increase the target area for the puncture needle penetrating the tracheoesophagal wall.

The tubular element **204** is provided with a proximal part **206** corresponding in shape and size to the lumen of the tube **201.** The proximal part may thus be inserted into the lumen of the tube **201.** When inserted into the lumen of the tube **201,** the lumen of the tubular element **204,** communicates with the lumen of the tube **201,** such that a guide wire may be inserted through the opening **205** and further passed proximally out of the pharynx protector and thereby out of the mouth of the patient.

On the outside of the proximal part **206** protrusions **207** extends outwardly from the proximal part **206.** The protrusions **207** correspond in size and shape to the through openings **203** in the tube **201.** These protrusions **207** may be adapted to securingly fit within the openings **203,** such that the tubular element **204** may be snap fitted to the tube **201** by insertion of the proximal part **206** through the distal end of the tube **201.** In this way, the tubular element **204** may be secured to the tube **201** and thereby the pharynx protector **200.**

In the distal end of the tubular element **204** a tip element **208** may be provided, such that the distal opening of the tubular element **204** is closed.

The outer surface of the tip element **208** may be rounded, such as semi-spherical or dome shaped. Such shape will facilitate insertion of the pharynx protector through the mouth of the patient, and also facilitate the travel through the oesophagus of the patient to the target site. The tip element **208** may also be provided with an axial opening, having a direction axial to the central axis of the tube **201** and the tubular element **204.** This axial opening may be used for inspection with a flexible endoscope. The axial opening may also be used if a guide wire or catheter is used for insertion of the pharynx protector, making it easier to introduce the pharynx protector in a difficult throat with strictures and other anatomical defects.

In another embodiment, according to Figs. 3a to 3c, a pharynx protector **300** comprises a flexible tube **301,** provided with a receiving portion **302** at the distal end thereof. The receiving portion **302** is a portion for receiving a puncture needle after said puncture needle has penetrated the tracheoesophageal wall. To facilitate use and decrease discomfort to the patient the tube **301** should have a flexability sufficient to exit the mouth of the patient while still extending down into oesophagus, without too strong bending having to be resisted by the body of the patient.

Close to a distal end of the flexible tube **301,** the pharynx protector **300** is provided with a receiving portion **302,** for receiving the puncture needle after said puncture needle has penetrated the tracheoesophageal wall.

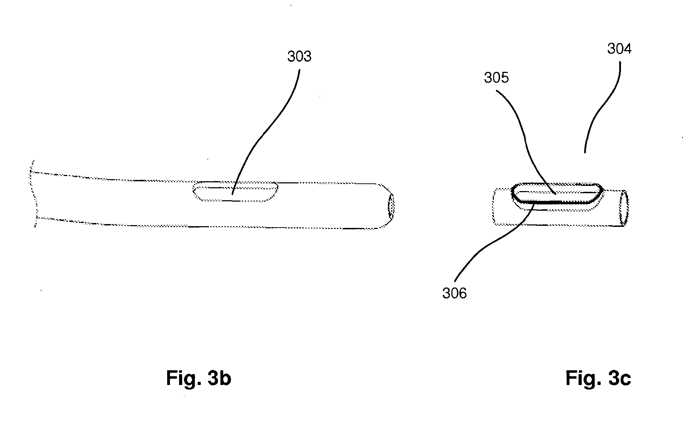

The receiving portion **302** comprises an opening **303** in the tubular wall of the flexible tube **301,** in accordance with Fig. 3b. The distal end of the flexible tube **301** is the end of the pharynx protector that is inserted through the mouth of the patient.

The receiving portion further comprises a rigid tubular element **304,** inserted into the lumen of the flexible tube **301,** in accordance with Figs. 3a and 3c. The rigidity of the tubular element **304** should be such that it a puncture needle cannot penetrate through the wall of the tubular element **304** during practical circumstances. Thus, the puncture needle cannot penetrate the back wall of the tubular element **304,** whereby the back wall of the oesophagus is protected.

The tubular element **304** has an opening **305** in the tubular wall thereof. The opening **305** corresponds in shape and size to the opening **303** in the tube **301.** The opening **305** may have a circumferential rim **306** on the outside surface of the tubular element **304.** This rim **306** may be adapted to securingly fit within the opening **303,** such that the tubular element **304** may be snap fitted to the tube **301** by insertion through the distal end of the tube **301.** In this way, the tubular element **304** may be secured to the tube **301** and thereby the pharynx protector **300.**

The lumen of the tubular element **304,** when inserted in the tube **301,** communicates with the lumen of the tube **301,** such that a guide wire may be inserted through the openings **303** and **305** and further passed proximally out of the pharynx protector and thereby out of the mouth of the patient.

The openings **303** and **305** may have an oval shape, which will increase the target area for the puncture needle penetrating the tracheoesophagal wall.

A tubular rounded tip may be formed by heat forming the tube **301,** whereby the tube shrinks, and forms a rounded tip shape, when further enclosing the tubular element **304.** Also, this may further improve the securement of the tubular element **304** to the tube **301** and thereby the pharynx protector **300.** The outer surface of the rounded tip may thus be rounded, such as semi-spherical or dome shaped. Such shape will facilitate insertion of the pharynx protector through the mouth of the patient, and also facilitate the travel through the oesophagus of the patient to the target site. The rounded tip may also be provided with an axial opening, having a direction axial to the central axis of the tube **301** and the tubular element **304.** This axial opening may be used for inspection with a flexible endoscope. The axial opening may also be used if a guide wire is used for insertion of the pharynx protector, making it easier to introduce the pharynx protector in a difficult throat with strictures and other anatomical defects.

The tube **101, 201, 301** is made of a flexible material for a non-traumatic insertion into the oesophagus, through the pharynx. A suitable material for the tube **101, 201, 301** is a thermoplastic elastomer. A suitable thermoplastic elastomer may be selected from the group comprising styrene block copolymers (SBS; SEBS), thermoplastic polyurethanes, thermoplastic copolyesters, thermoplastic copolyamides, elastomeric polyolefines, thermoplastic polyolefine elastomer blends,vulcanized thermoplastic polyolefine elastomers, plasticized polyvinyl chloride, blends between elastomers, such as SBS, SEBS, etc., and plastics, such as polyethylene, polypropylene, polystyrene, ethylene vinyl acetate, etc, or any combination of these. MEDIPRENE™ is an example of a suitable material, being a blend of SEBS, paraffinic oil, and polypropylene.

The tubular element **104, 204, 304** may be manufactured of a rigid material, such as rigid plastic material having a suitable hardness for the intended purpose, i.e. to ensure that the puncture needle does not penetrate the back wall of the head portion. A suitable material may for example be polyoxymethylene, polypropylene, a polyamide, and a polycarbonate.

The diameter of the axial opening in the tip **108, 208** or the rounded tip in Figs. 3a to 3c may suitably be selected to be in the interval of 2 to 8 mm, such as 4 to 6 mm.

The diameter of the flexible tube **101, 201, 301** may suitably be selected to be in the interval of 10 to 20 mm, such as 10 to 15 mm.

The length of the pharynx protector **100, 200, 300** may suitably be selected to be in the interval of 150 to 500 mm, such as 200 to 400 mm.

The diagonal of the openings **103, 105, 203, 205, 303, 305** may suitably be selected to be in the interval of 10 to 20 mm.

Of course, other additive attachment means, such as gluing, may be combined with the configuration of openings, rims, and protrusions, respectively, with regard the different embodiments disclosed herein.

The pharynx protector **100, 200, 300** is inserted into oesophagus of the patient, for example by swallowing. The opening **105, 205, 305** is positioned at the intended position of puncture. This can be assured by inserting a finger through the tracheostoma of the patient and palpating the outside of the oesophagus to thereby feel when the opening has the correct orientation. This can be achieved, since the oesophagus has flexible walls. Alternatively, a fiber endoscope may be inserted through the pharynx protector **100, 200, 300,** and illuminating the position of puncture with a light that penetrates the tracheal-oesophagal wall. After puncturing the intended position with a puncture needle, the guide wire is inserted through the puncture needle into the pharynx protector **100, 200, 300.** Thereafter, the puncture needle may be extracted and the guide wire may be pushed until it exits the mouth of the patient, whereafter the pharynx protector **100, 200, 300** may be extracted from the oesophagus of the patient.

Although the present invention has been described above with reference to specific embodiments, it is not intended to be limited to the specific form set forth herein. Rather, the invention is limited only by the accompanying claims and, other embodiments than the specific above are equally possible within the scope of these appended claims.

## Claims

1. A pharynx protector (100, 200, 300) for protecting the oesophagus and/or pharynx during performance of a secondary puncture in a laryngectomized patient, said pharynx protector having a proximal end and a distal end, said distal end being adapted to be inserted into the oesophagus of a patient, said pharynx protector comprising an elongated tubular part (101, 201, 301) and a distally located receiving portion (102, 202, 302) for receiving a puncture needle during laryngectomy
said tubular part being flexible, and
said receiving portion comprising; a first opening (105, 205, 305), communicating with the lumen of the tubular part, such that a guide wire may be inserted through the first opening and further passed proximally out of the pharynx protector; **characterized in that** a surface of the inner wall, having a normal passing through said opening, being of a rigid material.

2. The pharynx protector according to claim 1, wherein the distal end is rounded.

3. The pharynx protector according to claim 1 or 2, wherein the distal end has an axial opening.

4. The pharynx protector according to any of claims 1 to 3, wherein the receiving portion(105) comprises a rigid tubular element (104), positioned in the lumen of the tubular part (101), said tubular element (104) comprising the first opening (105) positioned underneath a second opening (103) in the tubular part (101).

5. The pharynx protector according to claim 4, wherein the first and the second openings are corresponding in size and shape.

6. The pharynx protector according to claim 4 or 5, wherein said tubular element (104) comprises a rim (106) on the outside surface of the tubular element (104) encircling the first opening (105), and fitting within the second opening (103) of the tubular part (101).

7. The pharynx protector according to any of claims 4 to 6, wherein the tubular element (104) comprises bars (107), extending distally and coaxially with the central axis of the tubular element (104), and a tip element (108) in the distal end thereof.

8. The pharynx protector according to any of claims 4 to 6, wherein the distal end has been heat formed, such that the distal end of the tubular part (301) is rounded.

9. The pharynx protector according to any of claims 1 to 3, wherein the receiving portion (202) comprises a rigid tubular element (204) comprising the first opening (205), said tubular element being positioned distally of the tubular part (201).

10. The pharynx protector according to claim 9, wherein a proximal part (206) of the tubular element (204) is inserted into the lumen of the tubular part (201), and protrusions (207), extending outwardly from the proximal part (206), are fitted within openings (203) in the distal end of the tubular part (201).

11. The pharynx protector according to claim 9 or 10, wherein the tubular element (204) is provided with a tip element (208) in the distal end thereof.

12. The pharynx protector according to any of the preceding claims, wherein the opening (105, 205, 305) has an oval shape.

13. The pharynx protector according to any of the preceding claims, wherein the tubular part (101, 201, 301) is made of a thermoplastic elastomer.

14. The pharynx protector according to claim 13, wherein the tubular part (101, 201, 301) is made of a material selected from the group consisting of styrene block copolymers (SBS; SEBS), thermoplastic polyurethanes, thermoplastic copolyesters, thermoplastic copolyamides, elastomeric polyolefines, thermoplastic polyolefine elastomer blends,vulcanized thermoplastic polyolefine elastomers, plasticized polyvinyl chloride, blends between elastomers, and plastics, or any combination of these.

15. The pharynx protector according to claim 14, wherein the tubular part (101, 201, 301) is made of a blend of SEBS, paraffinic oil, and polypropylene, and the rigid material is polyoxymethylene, polypropylene, polyamides, a polyamide, or a polycarbonate.

## Patentansprüche

1. Pharynx-Schutz (100, 200, 300) zum Schutz von Speiseröhre und/oder Pharynx während der Durchführung einer sekundären Punktion in einem laryngektomierten Patienten, wobei der Pharynx-Schutz ein proximales Ende und eine distales Ende aufweist, wobei das distale Ende zur Einführung in die Speiseröhre eines Patienten ausgelegt ist, wobei der Pharynx-Schutz einen langgestreckten schlauchförmigen Teil (101, 201, 301) und einen distal angeordneten Aufnahmeabschnitt (102, 202, 302) zur Aufnahme einer Punktionsnadel während der Laryngektomie aufweist,
wobei der schlauchförmige Teil flexibel ist, und
der Aufnahmeabschnitt Folgendes umfasst: eine erste Öffnung (105, 205, 305), die mit dem Lumen des schlauchförmigen Teils in Verbindung steht, so dass ein Führungsdraht durch die erste Öffnung eingeführt werden kann und weiter proximal aus dem Pharynx-Schutz heraus geführt werden kann; **dadurch gekennzeichnet, dass** eine Oberfläche der Innenwand, die normal durch die Öffnung geführt wird, aus einem starren Material besteht.

2. Pharynx-Schutz nach Anspruch 1, worin das distale Ende abgerundet ist.

3. Pharynx-Schutz nach Anspruch 1 oder 2, worin das distale Ende eine axiale Öffnung aufweist.

4. Pharynx-Schutz nach einem der Ansprüche 1 bis 3, worin der Aufnahmeabschnitt (105) ein starres schlauchförmiges Element (104) umfasst, das in dem Lumen des schlauchförmigen Teils (101) angeordnet ist, wobei das schlauchförmige Element (104) die erste Öffnung (105) umfasst, die unter einer zweiten Öffnung (103) in dem schlauchförmigen Teil (101) angeordnet ist.

5. Pharynx-Schutz nach Anspruch 4, worin die ersten und zweiten Öffnungen sich in Größe und Form entsprechen.

6. Pharynx-Schutz nach Anspruch 4 oder 5, worin das schlauchförmige Element (104) einen Rand (106) auf der Außenfläche des schlauchförmigen Elements (104) umfasst, der die erste Öffnung (105) umgibt, und in die zweite Öffnung (103) des schlauchförmigen Teils (101) passt.

7. Pharynx-Schutz nach einem der Ansprüche 4 bis 6, worin das schlauchförmige Element (104) Stäbe (107) umfasst, die sich distal und koaxial mit der Mittelachse des schlauchförmigen Elements (104) erstrecken, und ein Spitzenelement (108) in dem distalen Ende davon.

8. Pharynx-Schutz nach einem der Ansprüche 4 bis 6, worin das distale Ende warmverformt wurde, so dass das distale Ende des schlauchförmigen Teils (301) abgerundet ist.

9. Pharynx-Schutz nach einem der Ansprüche 1 bis 3, worin der Aufnahmeabschnitt (202) ein starres schlauchförmiges Element (204) umfasst, das die erste Öffnung (205) umfasst, wobei das schlauchförmige Element distal von dem schlauchförmigen Teil (201) angeordnet ist.

10. Pharynx-Schutz nach Anspruch 9, worin ein proximaler Teil (206) des schlauchförmigen Elements (204) in das Lumen des schlauchförmigen Teils (201) eingeführt ist und sich von dem proximalen Teil (206) nach außen erstreckende Vorsprünge (207) in Öffnungen (203) in dem distalen Ende des schlauchförmigen Teils (201) eingepasst sind.

11. Pharynx-Schutz nach Anspruch 9 oder 10, worin das schlauchförmige Element (204) mit einem Spitzenelement (208) in dem distalen Ende davon ausgestattet ist.

12. Pharynx-Schutz nach einem der vorherstehenden Ansprüche, worin die Öffnung (105, 205, 305) eine ovale Gestalt aufweist.

13. Pharynx-Schutz nach einem der vorhergehenden Ansprüche, worin der schlauchförmige Teil (101, 201, 301) aus einem thermoplastischen Elastomer gefertigt ist.

14. Pharynx-Schutz nach Anspruch 13, worin der schlauchförmige Teil (101, 201, 301) aus einem Material gefertigt ist, das aus der aus Styrol-Blockcopolymeren (SBS; SEBS), thermoplastischen Polyurethanen, thermoplastischen Copolyestern, thermoplastischen Copolyamiden, elastomeren Polyolefinen, thermoplastischen Polyolefin-Elastomer-Gemischen, vulkanisierten thermoplastischen Polyolefin-Elastomeren, Weich-Polyvinylchlorid, Gemischen zwischen Elastomeren und Kunststoffen oder einer beliebigen Kombination davon bestehenden Gruppe ausgewählt ist.

15. Pharynx-Schutz nach Anspruch 14, worin der schlauchförmige Teil (101, 201, 301) aus einem Gemisch von SEBS, paraffinischem Öl und Polypropylen besteht und das starre Material Polyoxymethylen, Polypropylen, Polyamide, ein Polyamid oder ein Polycarbonat ist.

## Revendications

1. Dispositif de protection du pharynx (100, 200, 300) destiné à protéger l'oesophage et/ou le pharynx durant la réalisation d'une ponction secondaire chez un patient laryngectomisé, ledit dispositif de protection du pharynx ayant une extrémité proximale et une extrémité distale, ladite extrémité distale étant conçue pour être insérée dans l'oesophage d'un patient, ledit dispositif de protection du pharynx comprenant une partie tubulaire allongée (101, 201, 301) et située distalement d'une partie de réception (102, 202, 302) destinée à recevoir une aiguille de ponction durant une laryngectomie,
ladite partie tubulaire étant flexible, et
ladite partie de réception comprenant : une première ouverture (105, 205, 305), communiquant avec la lumière de la partie tubulaire, de telle sorte qu'un fil-guide peut être inséré dans la première ouverture et peut en outre ressortir proximalement du dispositif de protection du pharynx ; **caractérisé en ce qu'**une surface de la paroi intérieure, ayant une ligne normale passant à travers ladite ouverture, est en un matériau rigide.

2. Dispositif de protection du pharynx selon la revendication 1, dans lequel l'extrémité distale est arrondie.

3. Dispositif de protection du pharynx selon la revendication 1 ou la revendication 2, dans lequel l'extrémité distale a une ouverture axiale.

4. Dispositif de protection du pharynx selon l'une quelconque des revendications 1 à 3, dans lequel la partie de réception (105) comprend un élément tubulaire rigide (104), positionné dans la lumière de la partie tubulaire (101), ledit élément tubulaire (104) comprenant la première ouverture (105) positionnée en dessous d'une seconde ouverture (103) dans la partie tubulaire (101).

5. Dispositif de protection du pharynx selon la revendication 4, dans lequel les première et seconde ouvertures ont une taille et une forme correspondantes.

6. Dispositif de protection du pharynx selon la revendication 4 ou la revendication 5, dans lequel ledit élément tubulaire (104) comprend un rebord (106) sur la surface extérieure de l'élément tubulaire (104) encerclant la première ouverture (105), et s'ajustant à l'intérieur de la seconde ouverture (103) de la partie tubulaire (101).

7. Dispositif de protection du pharynx selon l'une quelconque des revendications 4 à 6, dans lequel l'élément tubulaire (104) comprend des barres (107), s'entendant de manière distale et coaxiale avec l'axe central de l'élément tubulaire (104), et un élément de pointe (108) dans son extrémité distale.

8. Dispositif de protection du pharynx selon l'une quelconque des revendications 4 à 6, dans lequel l'extrémité distale a été thermoformée, de telle manière que l'extrémité distale de la partie tubulaire (301) est arrondie.

9. Dispositif de protection du pharynx selon l'une quelconque des revendications 1 à 3, dans lequel la partie de réception (202) comprend un élément tubulaire rigide (204) comprenant la première ouverture (205), ledit élément tubulaire étant positionné distalement de la partie tubulaire (201).

10. Dispositif de protection du pharynx selon la revendication 9, dans lequel une partie proximale (206) de l'élément tubulaire (204) est insérée dans la lumière de la partie tubulaire (201), et des protubérances (207), s'étendant vers l'extérieur à partir de la partie proximale (206), sont ajustées à l'intérieur des ouvertures (203) dans l'extrémité distale de la partie tubulaire (201).

11. Dispositif de protection du pharynx selon la revendication 9 ou la revendication 10, dans lequel l'élément tubulaire (204) est doté d'un élément de pointe (208) dans son extrémité distale.

12. Dispositif de protection du pharynx selon l'une quelconque des revendications précédentes, dans lequel l'ouverture (105, 205, 305) a une forme ovale.

13. Dispositif de protection du pharynx selon l'une quelconque des revendications précédentes, dans lequel la partie tubulaire (101, 201, 301) est en un élastomère thermoplastique.

14. Dispositif de protection du pharynx selon la revendication 13, dans lequel la partie tubulaire (101, 201, 301) est en un matériau sélectionné dans le groupe constitué de copolymères séquencés de styrène (SBS ; SEBS), de polyuréthanes thermoplastiques, de copolyesters thermoplastiques, de copolyamides thermoplastiques, de polyoléfines élastomères, de mélanges d'élastomères de polyoléfines thermoplastiques, d'élastomères de polyoléfines thermoplastiques vulcanisés, de chlorure de polyvinyle plastifié, de mélanges d'élastomères, et de plastiques, ou toute combinaison de ceux-ci.

15. Dispositif de protection du pharynx selon la revendication 14, dans lequel la partie tubulaire (101, 201, 301) est constituée d'un mélange de SEBS, d'huile paraffinique, et de polypropylène, et le matériau rigide est le polyoxyméthylène, le polypropylène, les polyamides, un polyamide, ou un polycarbonate.
